(19) Europäisches Patentamt
European Patent Office
Office européen des brevets

(11) **EP 4 653 472 A2**

(12) **EUROPEAN PATENT APPLICATION**

(43) Date of publication:
**26.11.2025 Bulletin 2025/48**

(21) Application number: **25178486.4**

(22) Date of filing: **23.05.2025**

(51) International Patent Classification (IPC):
**C08B 11/00** (2006.01)    **C08B 11/02** (2006.01)
**C08L 1/28** (2006.01)

(52) Cooperative Patent Classification (CPC):
**C08B 11/00; C08B 11/02; C08L 1/28**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **24.05.2024 JP 2024084479**

(71) Applicant: SHIN-ETSU CHEMICAL CO., LTD.
**Tokyo 100-0005 (JP)**

(72) Inventors:
• **MURAYAMA, Tomotaka**
  **NIIGATA, 942-8601 (JP)**
• **MIKI, Kentaro**
  **NIIGATA, 942-8601 (JP)**
• **KITAMURA, Akira**
  **NIIGATA, 942-8601 (JP)**
• **HIRAMA, Yasuyuki**
  **NIIGATA, 942-8601 (JP)**

(74) Representative: **Ipsilon**
  **Le Centralis**
  **63, avenue du Général Leclerc**
  **92340 Bourg-la-Reine (FR)**

(54) **LOW-SUBSTITUTED HYDROXYPROPYL CELLULOSE AND METHOD FOR PRODUCING SAME**

(57)    Provided is a method for producing low-substituted hydroxypropyl cellulose having high flowability and favorable compactibility. The method includes essentially the steps of: bringing a solution of alkali metal hydroxide into contact with a powder pulp to prepare alkali cellulose; allowing the alkali cellulose and propylene oxide to react with each other to obtain a reaction product; mixing the reaction product with water, as a solubilization step, without adding any acid; neutralizing the alkali metal hydroxide contained in the reaction product; washing, dewatering and drying the neutralized reaction product after being subjected to the neutralizing step to produce dried low-substituted hydroxypropyl cellulose; and pulverizing the dried low-substituted hydroxypropyl cellulose.

EP 4 653 472 A2

FIG.1

**Description**

BACKGROUND OF THE INVENTION

Field of the invention

The present invention relates to low-substituted hydroxypropyl cellulose and a method for producing the same.

Background art

A solid pharmaceutical preparation as a pharmaceutical product, healthy food or the like is designed to disintegrate when a disintegrant contained therein absorbs water and swells therein. Examples of such disintegrant include low-substituted hydroxypropyl cellulose, carboxymethyl cellulose and a calcium salt thereof, as well as starch and a derivative thereof. Among them, low-substituted hydroxypropyl cellulose (hereafter also referred to as "L-HPC") which serves as a nonionic disintegrant and a binder has been used widely in the field of pharmaceuticals.

A tablet is one of the dosage forms of the solid pharmaceutical and is formed by compressing powder into a predetermined shape, which is produced using a tableting machine. It is desired that L-HPC of high flowability be used for producing tablets in respect of preventing jamming of the hopper of the tableting machine for manufacturing the tablets.

JP-A-H11-322802 reports a method for producing L-HPC with high flowability, where the wood pulp is immersed in caustic soda and then squeezed to obtain alkali cellulose. The resultant alkali cellulose is subjected to an etherification reaction, and the resultant reaction product of this etherification reaction is solubilized in water containing no acid, and then the alkali contained therein is completely neutralized by acid.

SUMMARY OF THE INVENTION

Although the L-HPC obtained by the method described in JP-A-H11-322802 exhibits high flowability, it turned out that the L-HPC exhibits an inferior compactibility between L-HPC particles in the tablet when it is formed into tablets. Meanwhile, the issue with producing L-HPC with high compactibility is that it also reduces flowability. Flowability and compactibility are inherently incompatible, thus making it difficult to achieve both high flowability and favorable compactibility simultaneously.

The invention has been made to overcome the drawback of the above-described prior art. An object of the present invention is to provide a method for producing L-HPC with high flowability and favorable compactibility.

The inventors of the present invention diligently conducted a series of studies to address the aforementioned objectives. They surprisingly found that performing an etherification reaction using alkali cellulose-obtained by bringing an alkali metal hydroxide solution into contact with powder pulp-and then mixing the reaction product with water, without adding any acid in the solubilization step, results in L-HPC having favorable compactibility despite exhibiting high flowability, thus completing the present invention.

The present invention provides low-substituted hydroxypropyl cellulose and a method for producing the low-substituted hydroxy-propyl cellulose that are as defined below.

<1> A method for producing low-substituted hydroxypropyl cellulose, comprising the steps of:

bringing a solution of alkali metal hydroxide into contact with a powder pulp to prepare alkali cellulose;
allowing the alkali cellulose and propylene oxide to react with each other to obtain a reaction product;
mixing the reaction product with water, as a solubilization step, without adding any acid;
neutralizing the alkali metal hydroxide contained in the reaction product;
washing, dewatering, and drying the neutralized reaction product after being subjected to the neutralizing step to produce dried low-substituted hydroxypropyl cellulose; and
pulverizing the dried low-substituted hydroxypropyl cellulose.

<2> The method according to <1>, wherein the ratio of the mass of water used in the solubilization step to the mass of cellulose in the powder pulp is 2.0 to 3.5.

<3> The method according to <1> or <2>, wherein the solubilization step is performed at a mixing temperature of 30 to 40°C.

<4> Low-substituted hydroxypropyl cellulose that is in a form of particles, comprising:

fine particles having a length of fiber of less than 40 μm;
spherical particles having a length of 40 μm or more and consisting of

first spherical particles having an elongation, which is a ratio of a diameter of fiber to a length of fiber, of 0.5 or more, and
second spherical particles having

an elongation of less than 0.5,
an aspect ratio, which is a ratio of a minimum Feret diameter to a maximum Feret diameter, of 0.5 or more, and
a circularity, which is a ratio of a perimeter ($P_{EQPC}$) of a circle that has the same area as a projection area of a particle to a real perimeter ($P_{real}$) of the particle, of 0.7 or more; and

fibrous particles consisting of

long fibrous particles having a length of fiber of 200 μm or more and an elongation of less than 0.5, and consisting of

first long fibrous particles having an aspect ratio of less than 0.5, and
second long fibrous particles having an aspect ratio of 0.5 or more and a circularity of less than 0.7, and

short fibrous particles having a length of fiber of 40 μm or more and less than 200 μm and an elongation of less than 0.5, and consisting of

first short fibrous particles having an aspect ratio of less than 0.5, and
second short fibrous particles having an aspect ratio of 0.5 or more and a circularity of less than 0.7,

wherein the low-substituted hydroxypropyl cellulose have

a hydroxypropoxy group content of 5 to 16% by mass,
a volume fraction of the spherical particles of 60 to 90% relative to all of the particles, and
a volume fraction ratio of the second spherical particles to the first spherical particles of 0.90 to 1.35, and

wherein said form of particles is classified into the fine particles, the spherical particles, and the fibrous particles on a basis of dynamic image analysis.

The present invention may provide L-HPC having both high flowability and favorable compactibility. When the L-HPC obtained by the production method of the present invention is used for producing pharmaceutical tablets, the L-HPC as obtained by the production method according to the present invention exhibits high flowability, which therefore allows the powder to prevent itself from being jammed in the hopper. The L-HPC produced by the method of the present invention also exhibits high compactibility, allowing the L-HPC to be added in smaller amounts to the tablets, thus enabling the compactification of the tablets.

BRIEF DESCRIPTION OF THE DRAWINGS

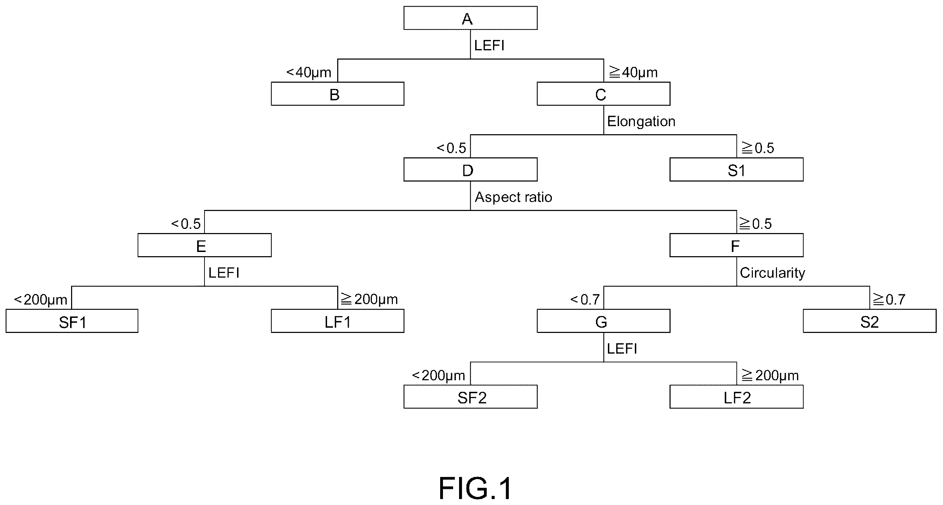

FIG. 1 shows a flowchart summarizing a method of categorizing "all particles" of L-HPC into seven types of particles: "Fine particles", "First long fibrous particles", "Second long fibrous particles", "First short fibrous particles", "Second short fibrous particles", "First spherical particles", and "Second spherical particles".

DETAILED DESCRIPTION OF THE INVENTION

The present invention is explained in greater detail hereinbelow.
The term "compactibility" as used herein refers to the characteristic value of the binding between L-HPCs when they are formed into a tablet. A higher level of compactibility indicates a greater likelihood of the L-HPC tablet maintaining its shape against external forces, while a lower level of compactibility indicates a lesser likelihood of the L-HPC tablet maintaining its shape against external forces.
The method for producing L-HPC according to the present invention essentially includes the steps of: bringing a solution of alkali metal hydroxide into contact with a powder pulp to prepare alkali cellulose; allowing the alkali cellulose and propylene oxide to react with each other to obtain a reaction product; mixing the reaction product with water, as a solubilization step, without adding any acid; neutralizing the alkali metal hydroxide contained in the reaction product; washing, dewatering, and drying the neutralized reaction product to produce dried low-substituted hydroxypropyl cellulose; and pulverizing the dried low-substituted hydroxypropyl cellulose.

[Step for preparing alkali cellulose]

A solution of alkali metal hydroxide is brought into contact with a powder pulp to prepare alkali cellulose.

Both the wood pulp and non-wood pulp such as linter pulp can be used as a raw material powder pulp but a wood originating pulp is preferable from the viewpoint of using a GMO (Genetically Modified Organism)-free material. As the tree species of the wood, coniferous trees such as pine, spruce, and tsuga; and hardwood (broad-leaved) trees such as eucalyptus and maple can be used. The powder pulp typically consists of cellulose and water. For this reason, the solid component in the pulp as used herein is calculated as a cellulose portion. The mass of the solid component in the pulp or cellulose portion can be calculated from the dry matter content determined in accordance with JIS P8203:2010: Pulp-Determination of dry matter content. The dry matter content is obtained as the ratio of the mass of the sample having been dried at $105 \pm 2°C$ and having reached constant mass to the mass of the sample before being dried, and is expressed as % by mass.

An alkali metal hydroxide solution of any type which is not particularly limited may be used so long as it allows pulp to make it into alkali cellulose but, from the viewpoint of economy, an aqueous solution of sodium hydroxide or potassium hydroxide is preferable. The concentration of the alkali metal hydroxide in the alkali metal hydroxide solution is preferably 20 to 60% by mass, more preferably 20 to 50% by mass from the viewpoints of the uniformity and reaction efficiency of the alkali cellulose.

It is preferred in terms of reaction efficiency of propylene oxide that the alkali cellulose contain alkali metal hydroxide in an amount of 5 to 35% by mass. The amount of alkali metal hydroxide contained in the alkali cellulose can be determined by neutralization titration of the alkali cellulose using an acid such as sulfuric acid of known concentration.

The powder pulp and the alkali metal hydroxide solution may be brought into contact at a temperature of preferably 20 to 80°C. The time for which the powder pulp and the alkali metal hydroxide solution are in contact with each other is preferably 5 to 120 minutes.

After the preparation of the alkali cellulose, the reaction vessel is preferably purged with an inert gas (preferably, with nitrogen or helium). Alternatively, the reaction vessel may be purged with an inert gas before preparing the alkali cellulose, and then the reaction vessel may be purged again with an inert gas after preparing the alkali cellulose.

[Step to obtain reaction product]

Next, a step of reacting the alkali cellulose prepared in the previous step with propylene oxide to obtain a reaction product is explained hereunder.

It is preferred that the amount of propylene oxide to be added thereinto be 0.05 to 0.5 parts by mass per 1 part by mass of anhydrous cellulose. The propylene oxide may be added using any one of the methods selected from a method of adding a predetermined amount of propylene oxide all at once, a method of adding a predetermined amount of propylene oxide in several batches, and a method of adding a predetermined amount of propylene oxide continuously. The reaction temperature for this step is preferably 40 to 80°C. The reaction time of this step is preferably 1 to 5 hours. This step is preferably performed under an inert gas (nitrogen or helium gas) atmosphere.

<Solubilization step>

A solubilization step of mixing the reaction product obtained in the previous step with water for solubilization without adding any acid is explained hereunder.

In this step, water and the reaction product obtained in the previous step are fed into a mixer and mixed to dissolve the L-HPC.

It is preferred in terms of obtaining L-HPC having both high flowability and favorable compactibility that the mass ratio of water to be mixed with the reaction product to the cellulose in the powder pulp used for producing the reaction product subjected to the solubilization step be 2.0 to 3.5, more preferably 2.5 to 3.5. A mass ratio of water to be mixed therewith below 2.0 may cause the L-HPC to have reduced flowability. Meanwhile, a mass ratio of water to be mixed therewith above 3.5 may cause the L-HPC to have reduced compactibility.

The mixing temperature is preferably 30 to 40°C. The mixing temperature as used herein refers to the jacket temperature of the mixer. The mixing time is preferably from 10 minutes to 5 hours.

Any type of mixer which is not particularly limited may be used so long as it allows the jacket temperature to be controlled, and examples thereof for use therein include a jacketed biaxial kneader and a jacketed reactor equipped with an internal stirrer.

<Neutralization step>

Next, a step of neutralizing the alkali metal hydroxide contained in the reaction product is explained hereunder.

In this step, an acid is added to the reaction product obtained in the solubilization step to neutralize the alkali metal hydroxide contained in the reaction product, thus precipitating L-HPC.

Examples of the acid for use therein include mineral acids, such as hydrochloric acid, sulfuric acid, and nitric acid, and organic acids such as formic acid and acetic acid, among which hydrochloric acid and acetic acid are preferred in terms of corrosiveness and toxicity.

The amount of acid for use therein is an equivalent amount required for neutralizing the alkali metal hydroxide contained in the alkali cellulose used for producing the reaction product. This acid may be used in the form of a mixture with water (aqueous solution).

The neutralization temperature is preferably 30 to 40°C. This step may be performed in a mixer that is the same as the one used for performing the solubilization step, and therefore the term "neutralization temperature" refers to a jacket temperature of a reactor or a mixer.

<Step of washing, dewatering, and drying>

Next, a step of washing, dewatering, and drying the reaction product obtained after the neutralization step to produce dried low-substituted hydroxypropyl cellulose is explained hereinbelow.

The washing and dewatering can be performed by, for example, allowing the precipitate to be in contact with water, and then removing water therefrom using a dehydrator.

It is preferred in terms of washability that the water for use in washing have a temperature of 50°C or higher. It is preferred in terms of economical perspective that the mass ratio of water for use in washing to the cellulose in the powder pulp used for producing the reaction product subjected to the solubilization step be 30 to 300.

Examples of the dehydrator for use therein include a batch-type centrifugal dehydrator and a press-type dehydrator. The batch-type centrifugal dehydrator may employ any level of centrifugal effect so long as it has sufficient dehydration capability but it is preferred from the viewpoint of productivity that the dehydrator demonstrate a centrifugal acceleration of 500 G or more.

Then, the L-HPC obtained by washing and dewatering is dried to produce dried low-substituted hydroxypropyl cellulose.

The drying may be carried out using a dryer. Examples of the dryer include a fluidized bed dryer, an air flow dryer, a box type dryer, a vibrating dryer, a natural convection type constant temperature dryer, a forced convection constant temperature dryer, and a shelf dryer. The drying temperature is preferably 60 to 120°C from the viewpoint of drying efficiency. The drying time is preferably 0.5 to 36 hours from the viewpoint of productivity.

<Pulverizing step>

A step of pulverizing the dried low-substituted hydroxypropyl cellulose is explained hereinbelow.

Pulverization can be carried out using a pulverizer. Examples of such pulverizer include an impact-type pulverizer, such as a hammer mill, an impact mill, and a Victory mill, and a compaction pulverizer, such as a roller mill and a ball mill. Among them, an impact-type pulverizer is preferred in terms of energy efficiency.

Moreover, it is preferred that the pulverized L-HPC be sieved to remove insufficiently pulverized coarse powder. A sieve having an opening size of preferably 45 to 250 $\mu$m, more preferably 75 to 150 $\mu$m can be used.

The L-HPC obtained by the production method according to the present invention is explained.

The hydroxypropoxy group content of L-HPC is 5.0 to 16.0% by mass, preferably 6.0 to 15.0% by mass, and more preferably 7.0 to 14.0% by mass. L-HPC having a hydroxypropoxy group content of less than 5.0% by mass may exhibit a low level of swelling property so that it leads to an insufficient level of disintegratability when it is used for a tablet. Meanwhile, L-HPC having a hydroxypropoxy group content of higher than 16.0% by mass may make the L-HPC soluble in water. The hydroxypropoxy group content of L-HPC can be measured by the assay described in "Low-Substituted Hydroxypropyl cellulose" of the Japanese Pharmacopoeia 18th Edition.

In this specification, the low-substituted hydroxypropyl cellulose is categorized into four types of particles: "Long fibrous particles", "Short fibrous particles", "Spherical particles" and "Fine particles". In addition, the long fibrous particles are further categorized into "First long fibrous particles" and "Second long fibrous particles", the short fibrous particles are further categorized into "First short fibrous particles", and "Second short fibrous particles", and the spherical particles are further categorized into "First spherical particles", and "Second spherical particles". FIG. 1 shows a flowchart summarizing a method of categorizing these.

A volume fraction of each type of particles in L-HPC can be calculated based on the dynamic-image analysis by measuring the shape parameters such as a length of fiber (LEFI), a diameter of fiber (DIFI), an elongation, an aspect ratio and a circularity. Dynamic image analysis is a method in which images of particles dispersed in a fluid such as a gas or a solvent are continuously photographed and are binarized to perform the analysis to determine the diameter and shape of the particles. The analysis can be performed by using, for example, a dynamic-image analysis type particle diameter distribution analyzer, QICPIC/R16 (manufactured by Sympatec GmbH).

All particles A can be categorized into: Particles C having a length of fiber (LEFI) of 40 $\mu$m or more; and fine particles B having a length of fiber of less than 40 $\mu$m. The LEFI is defined as the length of the longest direct path that connects one end to the other end of the particle within the contour of the particle. A QICPIC/R16 equipped with an M7 lens has a detection limit of 4.7 $\mu$m, and thus fails to detect a particle of an LEFI of less than 4.7 $\mu$m. However, the volume content of the particles having an LEFI of less than 4.7 $\mu$m is extremely small relative to that of the entire L-HPC, so it is negligible for the purposes of the invention.

The particles C having an LEFI of 40 $\mu$m or more are categorized into: First spherical particles (S1) having an elongation of 0.5 or more; and particles D having an elongation of less than 0.5, wherein the elongation is a ratio (DIFI/LEFI) of a diameter of the fiber (DIFI) to the LEFI of the particle. The DIFI is defined as the minor diameter of a particle, and is calculated by dividing the projection area of the particle by the sum of all lengths of the fiber branches of the particle.

The particles D having an LEFI of 40 $\mu$m or more and an elongation of less than 0.5 are categorized into: Particles E having an aspect ratio of less than 0.5; and Particles F having an aspect ratio of 0.5 or more, wherein the aspect ratio is a ratio ($F_{min}/F_{max}$) of the minimum Feret diameter ($F_{min}$) to the maximum Feret diameter ($F_{max}$). Each particle has an aspect ratio of more than 0 and less than or equal to 1. The Feret diameter is the distance between two parallel tangent lines that put the particle therebetween. The maximum Feret diameter ($F_{max}$) is the largest distance between two parallel tangent lines sandwiching the particle in consideration of all possible orientations by changing the directions from 0° to 180°, and the minimum Feret diameter ($F_{min}$) is the minimum distance between two parallel tangent lines sandwiching the particle in consideration of all possible orientations by changing the direction from 0° to 180°.

The particles E having an LEFI of 40 $\mu$m or more, an elongation of less than 0.5, and an aspect ratio of less than 0.5 are categorized into First long fibrous particles (LF1) having an LEFI of 200 $\mu$m or more and first short fibrous particles (SF1) having an LEFI of less than 200 $\mu$m.

The particles F having an LEFI of 40 $\mu$m or more, and an elongation of less than 0.5, and an aspect ratio of 0.5 or more are categorized into Second spherical particles (S2) having a circularity of 0.7 or more and particles G having a circularity of less than 0.7. The circularity refers to a ratio of the perimeter ($P_{EQPC}$) of a circle that has the same area as the projection area ($A_p$) of the particle to the real perimeter ($P_{real}$) of the particle, and is defined by the equation as defined below.

$$\text{Circularity} = P_{EQPC} / P_{real} = 2 \sqrt{\pi \cdot A_p} / P_{real}$$

Each particle has a circularity of more than 0 and less than or equal to 1. A particle having a smaller circularity has a more irregular shape. The EQPC is the diameter of a circle of an equal projection area, and is defined as the diameter of a circle that has the same area as the projection area of the particle, and is also referred to as Heywood diameter.

The particles G having an LEFI of 40 $\mu$m or more, an elongation of less than 0.5, an aspect ratio of 0.5 or more, and a circularity of less than 0.7 are categorized into Second long fibrous particles (LF2) having an LEFI of 200 $\mu$m or more and Second short fibrous particles (SF2) having an LEFI of less than 200 $\mu$m.

<Volume of fine particles>

The volume ($V_m$) of the fine particles in L-HPC can be calculated by the following equation, where each fine particle is assumed to be a sphere having a diameter of EQPC.

$$V_m = (\pi/6) \times (EQPC)^3 \times N_m$$

where $N_m$ is the number of fine particles in a sample, and EQPC is a median EQPC corresponding to the 50% cumulative value on a number-based cumulative particle diameter distribution curve of the fine particles.

<Volume of first long fibrous particles >

The volume ($V_{LF1}$) of the first long fibrous particles in L-HPC can be calculated by the following equation, where each first long fibrous particle is assumed to have the shape of cylinder with a bottom diameter of DIFI and a height of LEFI.

$$V_{LF1} = (\pi/4) \times (DIFI)^2 \times (LEFI) \times N_{LF1}$$

where $N_{LF1}$ is the number of first long fibrous particles in a sample, DIFI is a median DIFI corresponding to the 50% cumulative value on a number-based cumulative particle diameter distribution curve of the first long fibrous particles, and LEFI is a median LEFI corresponding to the 50% cumulative value on a number-based cumulative particle diameter distribution curve of the first long fibrous particles.

<Volume of second long fibrous particles >

The volume ($V_{LF2}$) of the second long fibrous particles in L-HPC can be calculated by the following equation, where each second long fibrous particle is assumed to have the shape of cylinder with a bottom diameter of DIFI and a height of LEFI.

$$V_{LF2} = (\pi/4) \times (DIFI)^2 \times (LEFI) \times N_{LF2}$$

where $N_{LF2}$ is the number of second long fibrous particles in a sample, DIFI is a median DIFI corresponding to the 50% cumulative value on a number-based cumulative particle diameter distribution curve of the second long fibrous particles, and LEFI is a median LEFI corresponding to the 50% cumulative value on a number-based cumulative particle diameter distribution curve of the second long fibrous particles.

<Volume of first short fibrous particles >

The volume ($V_{SF1}$) of the first short fibrous particles in L-HPC can be calculated by the following equation, where each first short fibrous particle is assumed to have the shape of cylinder with a bottom diameter of DIFI and a height of LEFI.

$$V_{SF1} = (\pi/4) \times (DIFI)^2 \times (LEFI) \times N_{SF1}$$

where $N_{SF1}$ is the number of first short fibrous particles in a sample, and DIFI is a median DIFI corresponding to the 50% cumulative value on a number-based cumulative particle diameter distribution curve of the first short fibrous particles and LEFI is a median LEFI corresponding to the 50% cumulative value on a number-based cumulative particle diameter distribution curve of the first short fibrous particles.

<Volume of second short fibrous particles>

The volume ($V_{SF2}$) of the second short fibrous particles in L-HPC can be calculated by the following equation, where each second short fibrous particle is assumed to have the shape of cylinder with a bottom diameter of DIFI and a height of LEFI.

$$V_{SF2} = (\pi/4) \times (DIFI)^2 \times (LEFI) \times N_{SF2}$$

where $N_{SF2}$ is the number of second short fibrous particles in a sample, and DIFI is a median DIFI corresponding to the 50% cumulative value on a number-based cumulative particle diameter distribution curve of the second short fibrous particles and LEFI is a median LEFI corresponding to the 50% cumulative value on a number-based cumulative particle diameter distribution curve of the second short fibrous particles.

<Volume of first spherical particles>

The volume ($V_{S1}$) of the first spherical particles in L-HPC can be calculated by the following equation, where each first spherical particle is assumed to be a sphere having a diameter of EQPC.

$$V_{S1} = (\pi/6) \times (EQPC)^3 \times N_{S1}$$

where $N_{S1}$ is the number of first spherical particles in a sample, and EQPC is a median EQPC corresponding to the 50% cumulative value on a number-based cumulative particle diameter distribution curve of the first spherical particles.

<Volume of second spherical particles>

The volume ($V_{S2}$) of the second spherical particles in L-HPC can be calculated by the following equation, where each second spherical particle is assumed to be a sphere having a diameter of EQPC.

$$V_{S2} = (\pi/6) \times (EQPC)^3 \times N_{S2}$$

where $N_{S2}$ is the number of second spherical particles in a sample, and EQPC is a median EQPC corresponding to the 50% cumulative value on a number-based cumulative particle diameter distribution curve of the second spherical particles.

<Volume of all particles>

The volume $V_{total}$ of all particles in L-HPC can be determined using the volumes $V_m$, $V_{LF1}$, $V_{LF2}$, $V_{SF1}$, $V_{SF2}$, $V_{S1}$, and $V_{S2}$ as defined above and based on a formula defined as:

$$V_{total} = V_m + V_{LF1} + V_{LF2} + V_{SF1} + V_{SF2} + V_{S1} + V_{S2}.$$

<Volume fractions of particles>

The volume fraction of each type of particle to all particles in L-HPC may be calculated using the volumes of $V_m$, $V_{LF1}$, $V_{LF2}$, $V_{SF1}$, $V_{SF2}$, $V_{S1}$, and $V_{S2}$ of the respective type of particles and the volume $V_{total}$ of all particles as defined above based on the formulae as defined below.

$$\text{Volume fraction of fine particles } R_m(\%) = V_m/V_{total} \times 100$$

$$\text{Volume fraction of first long fibrous particles } R_{LF1}(\%) = V_{LF1}/V_{total} \times 100$$

$$\text{Volume fraction of second long fibrous particles } R_{LF2}(\%) = V_{LF2}/V_{total} \times 100$$

$$\text{Volume fraction of first short fibrous particles } R_{SF1}(\%) = V_{SF1}/V_{total} \times 100$$

$$\text{Volume fraction of second short fibrous particles } R_{SF2}(\%) = V_{SF2}/V_{total} \times 100$$

$$\text{Volume fraction of first spherical particles } R_{S1}(\%) = V_{S1}/V_{total} \times 100$$

$$\text{Volume fraction of second spherical particles } R_{S2}(\%) = V_{S2}/V_{total} \times 100$$

It is preferred that the volume fraction of the spherical particles relative to the total volume of all particles be 60 to 90%, more preferably 66 to 88%, when all particles in the L-HPC are categorized into the fine particles, spherical particles consisting of the first and second spherical particles, and fibrous particles consisting of the first and second fibrous particles. The L-HPC having a volume fraction of the spherical particles to all particles below 60% results in unfavorable flowability, while the L-HPC having a volume fraction thereof exceeding 90% leads to insufficient compactibility.

Moreover, the L-HPC has a volume fraction ratio of the second spherical particles to the first spherical particles (second fibrous particles/first fibrous particles, namely $R_{S2}/R_{S1}$) of 0.90 to 1.35. The L-HPC having a volume fraction ratio of the second spherical particles to the first spherical particles (second fibrous particles/first fibrous particles) below 0.90 leads to insufficient compactibility, while the L-HPC having a volume fraction ratio thereof exceeding 1.35 results in unfavorable flowability.

It is preferred in terms of compactibility and flowability that the L-HPC have a volume-based median particle diameter determined by dry laser diffraction method of 10 to 100 $\mu$m, more preferably of 30 to 90 $\mu$m, and even more preferably of 40 to 70 $\mu$m. The term "median particle diameter" as used herein refers to a diameter corresponding to the 50% cumulative value on a volume-based cumulative distribution curve of the particles, which can be measured using, for example, a laser diffraction particle size analyzer of Mastersizer 3000 (manufactured by Malvern Panalytical Ltd.)

It is preferred in terms of flowability that the L-HPC has a uniformity coefficient of 2.0 to 2.9, more preferably 2.1 to 2.8, and even more preferably 2.2 to 2.7. The uniformity coefficient can be calculated based on the measurements of a

diameter ($D_{60}$) corresponding to the 60% cumulative value on a volume-based cumulative particle diameter distribution curve and a diameter ($D_{10}$) corresponding to the 10% cumulative value on a volume-based cumulative particle diameter distribution curve in accordance with a formula as defined below:

$$\text{Uniformity coefficient} = D_{60}/D_{10}.$$

These $D_{60}$ and $D_{10}$ can be measured using, for example, a laser diffraction particle size analyzer of Mastersizer 3000 (manufactured by Malvern Panalytical Ltd). A powder with a smaller uniformity coefficient is regarded as having better flowability.

It is preferred in terms of flowability that the degree of compression of L-HPC be 20 to 29%, more preferably 21 to 28%, and even more preferably 22 to 27%. The degree of compression can be calculated from the values of Tapped bulk density ($BD_T$) and Loose bulk density ($BD_L$) based on a formula defined as:

$$\text{Degree of compression } (\%) = \{(BD_T - BD_L)/BD_T\} \times 100.$$

A powder with a smaller degree of compression is considered to have excellent flowability.

The term "Loose bulk density" refers to a bulk density in a loosely packed state, and is measured using, for example, a powder characteristics tester of POWDER TESTER PT-S type (manufactured by Hosokawa Micron Corporation) by which a material is uniformly supplied into a cylindrical container (whose material is stainless steel) having a diameter of 5.05 cm and a height of 5.05 cm (volume: 100 ml) from (23 cm) above the cylindrical container, and the supplied material is leveled at the top face of the container for weighing.

The term "tapped bulk density" refers to a bulk density in the state where a sample is closely packed into the container by tapping. The term "tapping" refers to an operation in which a cylindrical container filled with a sample is repeatedly dropped from a predetermined height to apply a light impact to the bottom of the container, thereby closely packing the sample in the container. Specifically, as is the case of measuring the "loose bulk density", a sample is supplied into the cylindrical container, and the supplied sample is leveled at the top face of the container for weighing, and then the cylindrical container is capped at the top with a cap, after which the cap is filled with the sample up to the upper edge of the cap, and subjected to 180 times of tapping from a tap height of 1.8 cm. After the tapping has been completed, the cap is removed from the container, and then the sample is leveled at the top face of the container for weighing. The bulk density of this state is determined as the tapped bulk density.

It is preferred in terms of flowability that the L-HPC have an angle of repose of 20 to 55°, more preferably 25 to 50°, and even more preferably 30 to 45°. For example, the angle of repose can be determined using a powder characteristic tester of PT-S (manufactured by Hosokawa Micron Corporation) by which a powdery sample is fed onto a round table having a diameter of 80 mm from a height of 75 mm, and then an angle between the piled powder and the table is measured. A powder having a smaller angle of repose is considered to have excellent flowability.

It is preferred in terms of flowability that the L-HPC have an angle of spatula of 40 to 60°, more preferably 45 to 55°. The angle of spatula can be measured using, for example, a powder characteristics tester of POWDER TESTER PT-S type (manufactured by Hosokawa Micron Corporation). Specifically, a metallic spatula with a 22 mm width is gently lifted up out of a layer filled with powder, and the angle between the powder surface left on the spatula and the spatula is measured. Then, an impact is applied to the spatula, and the angle between the powder surface left on the spatula and the spatula is measured again. The angle of the spatula may be determined based on the above values using the formula as defined below:

$$\text{Angle of spatula} = (\text{Angle prior to applying impact} + \text{Angle after applying impact})/2$$

A powder with a smaller angle of spatula is considered to have excellent flowability.

The L-HPC has a flowability index of preferably 70 or higher. The flowability index refers to an index for determining flowability and was proposed by Carr (See, R. L. Carr, Chem. Eng, 72, Jan.18, 163, Feb.1, 69(1965), 76 Oct.13, 7(1969)). A detailed description thereof is given in "An Illustrated Explanation of Powder Properties (revised and enlarged edition)" (edited by the Japanese Society of Powder Technology and the Japanese Association of Powder Process Industry and Engineering, Nikkei Technical Books, 1985), page 151. This flowability index can be determined by measuring the uniformity coefficient, the degree of compression, the angle of repose, and the angle of spatula as defined above, calculating the respective indices from these measured values, and summing them up. A powder with a larger flowability index has a higher level of flowability.

It is preferred in terms of reducing the additive amount of L-HPC in a pharmaceutical tablet that the L-HPC have a compactibility of 85N or more.

The compactibility of the L-HPC according to the present invention may be determined by a process including: storing L-HPC in a desiccator (relative humidity: about 11%) of 25 °C containing a saturated aqueous solution of lithium chloride for one week to control the loss on drying (water content) to 2.8 to 4.0% by mass; compressing the stored L-HPC to form it into a 450 mg tablet at a tableting pressure of 10 kN (about 88.5 MPa) by using a table top type tablet press (for example, HANDTAB 200 (manufactured by ICHIHASHI SEIKI KYOTO JAPAN) equipped with a round and flat punch having a diameter of 12 mm to produce tablets); and determining the hardness of the tablet as the maximum strength at break when a load was applied to the tablet at a rate of 1 mm/sec in the tablet diameter direction until the tablet is broken by using a tablet hardness tester (for example, TBH-125 (manufactured by ERWEKA GmbH)).

The loss on drying (water content) of L-HPC can be determined by the method described in section 2.41 "Loss on Drying test" in "General Tests" of the Japanese Pharmacopoeia 18th Edition.

WORKING EXAMPLES

The present invention is explained in detail with reference to working and comparative examples. The present invention should not be limited in any way to the following examples.

Working Example 1

8402 g of powder pulp (8000 g of cellulose portion) was supplied into a 150L reactor equipped with an internal stirrer and the inside of the reactor was thoroughly purged with nitrogen by reducing the pressure and sealing the reactor with nitrogen. After the nitrogen replacement, 6,300 g of a 35% by mass aqueous sodium hydroxide solution was supplied into the reactor and stirred for 5 minutes at an internal temperature of 60°C to obtain alkali cellulose containing 15% by mass of sodium hydroxide (the mass ratio of sodium hydroxide to anhydrous cellulose in the alkali cellulose was 0.276).

After that, this reactor was thoroughly purged with nitrogen by reducing the pressure and sealing the reactor with nitrogen, and then 1,504 g of propylene oxide was supplied thereinto to make it react with each other while stirring the same for 75 minutes at an internal temperature of 60°C, resulting in 16,206 g of reaction product.

Then, 671 g of water at 35°C was put into a 5L kneader having a twin-shaft stirrer, and 680 g of the reaction product (333.5 g of anhydrous cellulose portion) was dispersed therein, and then mixed for 30 minutes at a jacket temperature of 35°C to dissolve the L-HPC. The amount of water used in the solubilization step was such that the mass ratio of water to cellulose in the powder pulp was 2.0.

Next, 420.4 g of a 33% by mass acetic acid aqueous solution was put into the kneader whose jacket temperature was maintained at 35°C to completely neutralize the sodium hydroxide contained in the reaction product, thus precipitating crude L-HPC.

The whole amount of the resultant crude L-HPC was dispersed in 15,000 g of hot water at 90°C, and washed using a batch type centrifugal dehydrator under the condition of rotation speed at 2,000 rpm and then dewatered. After that, the whole amount of the resultant dewatered product was dispersed again in 15,000 g of hot water at 90°C, and washed using the batch type centrifugal dehydrator under the condition of rotation speed at 2,500 rpm, and then dewatered. The resultant dewatered product was dried for 18 hours at 80°C using a shelf dryer, and the dried product was pulverized by an impact-type pulverizer (Victory mill VP-1 manufactured by Hosokawa Micron Corporation) and then sieved through a sieve having an opening size of 91 $\mu$m to obtain L-HPC.

The obtained L-HPC underwent the measurement of the hydroxypropoxy group content, and was measured for the median particle diameter, the uniformity coefficient, the degree of compression, the angle of repose, the angle of spatula, the volume fractions of particles (for the first long fibrous particles, second long fibrous particles, first short fibrous particles, second short fibrous particles, first spherical particles, second spherical particles, and fine particles), the flowability index, and the compactibility in the manners as described below. The flowability index was calculated from the uniformity coefficient, the degree of compression, the angle of repose, and the angle of spatula. The results are as shown in Table 1.

<Measurement of median particle diameter>

As for the median particle diameter, a diameter corresponding to the 50% cumulative value on the volume-based cumulative particle diameter distribution curve was measured under conditions of a dispersion pressure of 2 bar and a scattering intensity of 2 to 10% by the dry method according to the Fraunhofer diffraction theory using a laser diffraction particle size analyzer of Mastersizer 3000 (manufactured by Malvern Panalytical Ltd).

<Measurement of uniformity coefficient>

As for the uniformity coefficient, a diameter ($D_{60}$) corresponding to the 60% cumulative value of the volume-based cumulative particle diameter distribution curve and a diameter ($D_{10}$) corresponding to the 10% cumulative value of the volume-based cumulative particle diameter distribution curve were measured under conditions of a dispersion pressure of 2 bar and a scattering intensity of 2 to 10% by the dry method according to the Fraunhofer diffraction theory using a laser diffraction particle size analyzer of Mastersizer 3000 (manufactured by Malvern Panalytical Ltd), and the uniformity coefficient was determined based on the following formula:

$$\text{Uniformity coefficient} = D_{60}/D_{10}$$

<Measurement of degree of compression>

The degree of compression was calculated from the values of Tapped bulk density ($BD_T$) and Loose bulk density ($BD_L$) based on a formula defined as:

Degree of compression (%) = [(Tapped bulk density - Loose bulk density)/Tapped bulk density} $\times$ 100.

The term "Loose bulk density" refers to a bulk density in a loosely packed state, and was measured using, for example, a powder characteristics tester of POWDER TESTER PT-S type (manufactured by Hosokawa Micron Corporation) by which a material is uniformly supplied onto a cylindrical container (whose material is stainless steel) having a diameter of 5.05 cm and a height of 5.05 cm (volume: 100 ml) from (23 cm) above the cylindrical container, and the supplied material is leveled at the top face of the container for weighing.

The term "tapped bulk density" refers to a bulk density in the state where a sample is closely packed into the container by tapping. The term "tapping" refers to an operation in which a cylindrical container filled with a sample is repeatedly dropped from a predetermined height to apply a light impact to the bottom of the container, thereby closely packing the sample in the container. Specifically, as is the case of measuring the "loose bulk density", a sample was supplied into the cylindrical container, and the supplied sample was leveled at the top face of the container for weighing, and then the cylindrical container was capped at the top with a cap, after which the cap was filled with the sample up to the upper edge of the cap, and subjected to 180 times of tapping from a tap height of 1.8 cm. After the tapping had been completed, the cap was removed from the container, and then the sample was leveled at the top face of the container for weighing. The bulk density of this state was determined as the tapped bulk density.

<Measurement of angle of repose>

The angle of repose was determined using a powder characteristics tester of POWDER TESTER PT-S type (manufactured by Hosokawa Micron Corporation) by which a powdery sample was fed onto a round table having a diameter of 80 mm from a height of 75 mm, and then the angle between the piled powder and the table was measured.

<Measurement of angle of spatula >

The angle of spatula was measured using a powder characteristics tester of POWDER TESTER PT-S type (manufactured by Hosokawa Micron Corporation) by which a metallic spatula with a 22mm width was gently lifted up out of a layer filled with powder, the angle between the powder surface left on the spatula and the spatula was measured, and then an impact was applied to the spatula, followed by measuring the angle between the powder surface left on the spatula and the spatula again. The angle of the spatula was determined based on the above values using the formula as defined below:

$$\text{Angle of spatula} = (\text{Angle prior to applying impact} + \text{Angle after applying impact})/2$$

<Measurement of volume fraction of each type of particles>

The volume fraction of each type of particles, which are first long fibrous particles, second long fibrous particles, first short fibrous particles, second short fibrous particles, first spherical particles, second spherical particles, and fine particles, was determined using a dynamic image analysis type particle diameter distribution analyzer QICPIC/R16 (manufactured by Sympatec GmbH) equipped with a quantitative feeder VIBRI/L, an air flow type disperser RODOS/L and an M7 lens under the conditions of a frame rate of 500 Hz, an injector of 4 mm, a dispersion pressure of 1 bar. The graphics of the imaged particles were analyzed by analysis software WINDOX5 Version 5.9.1.1 to determine the number-based median EQPC, the number-based median LEFI, the number-based median DIFI, the elongation, the aspect ratio, and the circularity with respect to each type of particles. The volume fraction of each type of particle was calculated by the above equation based on the measured values. It is noted that M7 was used as the division of analysis.

<Calculation of flowability index>

The flowability index was calculated by measuring the above-defined uniformity coefficient, the degree of compression, the angle of repose, and the angle of spatula, calculating the respective indices from these measured values, and then summing them up. A powder with a larger flowability index is considered to have excellent flowability. In the present invention, a flowability index higher than 70 was regarded as one having favorable flowability.

<Measurement of compactibility>

The compactibility was determined by a process including: storing L-HPC in a desiccator (relative humidity: about 11%) of 25°C containing a saturated aqueous solution of lithium chloride for one week to control the loss on drying (water content) to 2.8 to 4.0% by mass; compressing the stored L-HPC to form it into a 450 mg tablet at a tableting pressure of 10 kN (about 88.5 MPa) by using a table top type tablet forming press of HANDTAB 200 (manufactured by ICHIHASHI SEIKI KYOTO JAPAN) equipped with a round and flat punch having a diameter of 12 mm to produce tablets; and determining the hardness of the tablet as the maximum strength at break when a load was applied to the tablet at a rate of 1 mm/sec in the tablet diameter direction until the tablet was broken by using a tablet hardness tester of TBH-125 (manufactured by ERWEKA GmbH). A powder with a larger value of maximum strength at break was regarded as one having more favorable compactibility. In the present invention, a maximum strength at break higher than 85N was regarded as one having favorable compactibility.

Working Example 2

L-HPC was produced in the same manner as in Working Example 1 except that an amount by mass of water used in the solubilization step relative to an amount by mass of cellulose in the powder pulp was changed to 2.5. The produced L-HPC was measured for the hydroxypropoxy group content, the median particle diameter, the uniformity coefficient, the degree of compression, the angle of repose, the angle of spatula, the volume fractions of particles (for the first long fibrous particles, second long fibrous particles, first short fibrous particles, second short fibrous particles, first spherical particles, second spherical particles, and fine particles), the flowability index, and the compactibility. The flowability index was calculated from the uniformity coefficient, the degree of compression, the angle of repose, and the angle of spatula. The results are as shown in Table 1.

Working Example 3

L-HPC was produced in the same manner as in Working Example 1 except that an amount by mass of water used in the solubilization step relative to an amount by mass of cellulose in the powder pulp was changed to 3.0. The produced L-HPC was measured for the hydroxypropoxy group content, the median particle diameter, the uniformity coefficient, the degree of compression, the angle of repose, the angle of spatula, the volume fractions of particles (for the first long fibrous particles, second long fibrous particles, first short fibrous particles, second short fibrous particles, first spherical particles, second spherical particles, and fine particles), the flowability index, and the compactibility. The flowability index was calculated from the uniformity coefficient, the degree of compression, the angle of repose, and the angle of spatula. The results are as shown in Table 1.

Working Example 4

L-HPC was produced in the same manner as in Working Example 1 except that an amount by mass of water used in the solubilization step relative to an amount by mass of cellulose in the powder pulp was changed to 3.5. The produced L-HPC was measured for the hydroxypropoxy group content, the median particle diameter, the uniformity coefficient, the degree of compression, the angle of repose, the angle of spatula, the volume fractions of particles (for the first long fibrous particles, second long fibrous particles, first short fibrous particles, second short fibrous particles, first spherical particles, second spherical particles, and fine particles), the flowability index, and the compactibility. The flowability index was calculated from the uniformity coefficient, the degree of compression, the angle of repose, and the angle of spatula. The results are as shown in Table 1.

Comparative Example 1

L-HPC was produced in the same manner as in Working Example 2 except that 84.1 g of a 33% by mass acetic acid aqueous solution was added to water in the solubilization step before dispersing the reaction product in water. The produced L-HPC was measured for the hydroxypropoxy group content, the median particle diameter, the uniformity coefficient, the degree of compression, the angle of repose, the angle of spatula, the volume fractions of particles (for the first long fibrous particles, second long fibrous particles, first short fibrous particles, second short fibrous particles, first spherical particles, second spherical particles, and fine particles), the flowability index, and the compactibility. The flowability index was calculated from the uniformity coefficient, the degree of compression, the angle of repose, and the angle of spatula. The results are as shown in Table 1.

Comparative Example 2

Sheet pulp (having a water content of 7.27% by mass) was soaked in a 35% by mass sodium hydroxide aqueous solution at 20°C, and then pressed to remove the excess aqueous sodium hydroxide solution to prepare a sheet of alkali cellulose containing 16.9% by mass of sodium hydroxide (in which the mass ratio of sodium hydroxide relative to anhydrous cellulose in alkali cellulose was 0.353). This sheet of alkali cellulose was shredded by a slitter cutter to obtain alkali cellulose chips.

Then, 625.9 g of these alkali cellulose chips (300 g of cellulose portion) was put into a rotary reactor, and the inside of the reactor was thoroughly purged with nitrogen by reducing the pressure and sealing the reactor with nitrogen, and then 68.7 g of propylene oxide was supplied thereinto to make it react with each other while stirring the same for 180 minutes at an internal temperature of 50°C, resulting in 694.6 g of reaction product.

Then, 734.3 g of water at 35°C was put into a 5L kneader having a twin-shaft stirrer, and 680 g of the reaction product (293.7 g of anhydrous cellulose portion) was dispersed therein, and then mixed for 30 minutes at a jacket temperature of 35°C to dissolve the L-HPC. The amount of water used in the solubilization step was such that the mass ratio of water to cellulose in the sheet pulp was 2.5.

Next, 471.0 g of a 33% by mass acetic acid aqueous solution was put into the kneader whose jacket temperature was maintained at 35°C to completely neutralize the sodium hydroxide contained in the reaction product, thus precipitating crude L-HPC.

The washing, dewatering, and drying steps and the subsequent steps thereafter were carried out in the same manner as in Working Example 1 to thereby obtain L-HPC. The obtained L-HPC was measured for the hydroxypropoxy group content, the median particle diameter, the uniformity coefficient, the degree of compression, the angle of repose, the angle of spatula, the volume fractions of particles (for the first long fibrous particles, second long fibrous particles, first short fibrous particles, second short fibrous particles, first spherical particles, second spherical particles, and fine particles), the flowability index, and the compactibility. The flowability index was calculated from the uniformity coefficient, the degree of compression, the angle of repose, and the angle of spatula. The results are as shown in Table 1.

Comparative Example 3

Sheet pulp (having a water content of 8.86% by mass) was soaked in a 43% by mass sodium hydroxide aqueous solution at 33.5°C, and then pressed to remove the excess aqueous sodium hydroxide solution to prepare a sheet of alkali cellulose containing 24% by mass of sodium hydroxide (in which the mass ratio of sodium hydroxide relative to anhydrous cellulose in alkali cellulose was 0.594). This sheet of alkali cellulose was shredded by a slitter cutter to obtain alkali cellulose chips.

Then, 495.8 g of these alkali cellulose chips (200 g of cellulose portion) was put into a rotary reactor and the reaction was carried out in the same manner as in Comparative Example 2 to obtain 551.8 g of reaction product.

Then, 1432.8 g of water at 33.5°C was put into a 5L kneader having a twin-shaft stirrer, and 549.3 g of the reaction product (199.0 g of anhydrous cellulose portion) was dispersed therein, and then mixed for 70 minutes at a jacket temperature of 33.5°C to dissolve the L-HPC. The amount of water used in the solubilization step was such that the mass ratio of water to cellulose in the sheet pulp was 7.2.

Next, 537.4 g of a 33% by mass acetic acid aqueous solution was put into the kneader whose jacket temperature was maintained at 33.5°C to completely neutralize the sodium hydroxide contained in the reaction product, thus precipitating crude L-HPC.

The washing, dewatering, and drying steps and the subsequent steps thereafter were carried out in the same manner as in Working Example 1 to thereby obtain L-HPC. The obtained L-HPC was measured for the hydroxypropoxy group content, the median particle diameter, the uniformity coefficient, the degree of compression, the angle of repose, the angle of spatula, the volume fractions of particles (for the first long fibrous particles, second long fibrous particles, first short fibrous particles, second short fibrous particles, first spherical particles, second spherical particles, and fine particles), the flowability index, and the compactibility. The flowability index was calculated from the uniformity coefficient, the degree of compression, the angle of repose, and the angle of spatula. The results are as shown in Table 1.

Table 1

| | | Production condition | | | | | | |
|---|---|---|---|---|---|---|---|---|
| | Pulp shape | Percentage by mass of sodium hydroxide in alkali cellulose | Weight ratio of water to cellulose in pulp in solubilization step | Percentage of acetic acid added in solubilization step with respect to the neutralization amount | Mixing time in solubilization step | Jacket temperature in solubilization step | Hydroxy propoxy group content |
| | [-] | [%] | [WR] | [%] | [min] | [°C] | [%] |
| Working example 1 | Powder | 15 | 2.0 | 0 | 30 | 35 | 11.2 |
| Working example 2 | Powder | 15 | 2.5 | 0 | 30 | 35 | 11.2 |
| Working example 3 | Powder | 15 | 3.0 | 0 | 30 | 35 | 11.2 |
| Working example 4 | Powder | 15 | 3.5 | 0 | 30 | 35 | 11.2 |
| Comparative example 1 | Powder | 15 | 2.5 | 20 | 30 | 35 | 11.2 |
| Comparative example 2 | Chip | 16.9 | 2.5 | 0 | 30 | 35 | 11.2 |
| Comparative example 3 | Chip | 24 | 7.2 | 0 | 70 | 33.5 | 10.9 |

| | Powder physical properties | | | | |
|---|---|---|---|---|---|
| | Median particle diameter | Uniformity coefficient | Degree of compression | Angle of repose | Angle of spatula |
| | [μm] | [-] | [%] | [°] | [°] |
| Working example 1 | 73.5 | 2.6 | 26.0 | 41.3 | 54.4 |
| Working example 2 | 71.8 | 2.4 | 24.1 | 37.2 | 54.0 |
| Working example 3 | 73.7 | 2.3 | 24.9 | 34.1 | 53.7 |
| Working example 4 | 73.9 | 2.3 | 23.3 | 33.8 | 53.4 |
| Comparative example 1 | 68.6 | 2.8 | 30.2 | 40.2 | 54.2 |
| Comparative example 2 | 74.6 | 2.8 | 32.1 | 43.2 | 64.8 |
| Comparative example 3 | 54.4 | 3.1 | 34.0 | 44.0 | 58.1 |

| | Powder physical properties | | | | | | | | | |
|---|---|---|---|---|---|---|---|---|---|---|
| | Volume fraction | | | | | | | Volume fraction of second spherical particles relative to first spherical particles | Flowability index | Compactibility |
| | First long fibrous particle | Second long fibrous particle | First short fibrous particle | Second short fibrous particle | First spherical particle | Second spherical particle | Fine particles | | | |
| | [%] | [%] | [%] | [%] | [%] | [%] | [%] | [-] | [-] | [N] |
| Working example 1 | 8.1 | 1.7 | 16.9 | 3.0 | 30.2 | 39.4 | 0.7 | 1.30 | 70.5 | 131 |
| Working example 2 | 4.2 | 1.0 | 12.4 | 2.1 | 37.0 | 42.5 | 0.8 | 1.15 | 73.0 | 89 |
| Working example 3 | 2.8 | 0.9 | 9.6 | 1.8 | 39.6 | 44.5 | 0.8 | 1.12 | 75.0 | 103 |
| Working example 4 | 2.1 | 0.7 | 7.8 | 1.5 | 45.2 | 41.9 | 0.8 | 0.93 | 76.0 | 98 |
| Comparative example 1 | 7.7 | 2.1 | 19.4 | 3.2 | 22.2 | 43.9 | 1.6 | 1.97 | 68.5 | 141 |
| Comparative example 2 | 11.7 | 2.1 | 15.9 | 2.3 | 27.1 | 39.8 | 1.1 | 1.47 | 60.5 | 133 |
| Comparative example 3 | 11.6 | 2.5 | 8.7 | 2.0 | 40.8 | 29.8 | 4.5 | 0.73 | 62.0 | 47 |

The L-HPCs of Working Examples 1 to 4 exhibited both favorable flowability and compactibility. In contrast, the L-HPCs of Comparative Examples 1 and 2 exhibited unfavorable flowability, and the L-HPC of Comparative Example 3 exhibited both unfavorable flowability and compactibility.

These results indicate that the L-HPCs of Working Examples 1 to 4, which were produced by employing powder pulps as raw material pulps and being mixed with water without adding any acid in the solubilizing step, resulted in a higher number of fibrous L-HPCs being lost and an increased proportion of spherical particles. It is assumed that this, in turn, led to favorable flowability. In addition, the L-HPCs of Working Examples 1 to 4 kept an overall proportion of the spherical particles compared to those of Comparative Examples 1 to 3, and exhibited increased volume fraction ratios of the second spherical particles to the first spherical particles. The second spherical particles have a more fibrous shape compared to the first spherical particles. For this reason, it is presumed that the working examples with an increased volume fraction of second spherical particles relative to the first spherical particles exhibited favorable compactibility as well. Comparative Example 2 used pulp chips as the raw material pulp. For this reason, it is also presumed that this led to an undissolved reaction product of the chips remaining in the solubilization step, despite the same amount of water mixed in the solubilization step as the one of Working Example 2, which, in turn, reduced the fraction of the spherical particles and lowered the flowability. Moreover, the results in Working examples 1 to 4 demonstrated that increasing the amount of water mixed in the solubilization step is more likely to enhance flowability, while reducing the amount of water is more likely to enhance compactibility.

**Claims**

1.  A method for producing low-substituted hydroxypropyl cellulose, comprising the steps of:

    bringing a solution of alkali metal hydroxide into contact with a powder pulp to prepare alkali cellulose;
    allowing the alkali cellulose and propylene oxide to react with each other to obtain a reaction product;
    mixing the reaction product with water, as a solubilization step, without adding any acid;
    neutralizing the alkali metal hydroxide contained in the reaction product;
    washing, dewatering, and drying the neutralized reaction product after being subjected to the neutralizing step to produce dried low-substituted hydroxypropyl cellulose; and
    pulverizing the dried low-substituted hydroxypropyl cellulose.

2.  The method according to claim 1, wherein the ratio of the mass of water used in the solubilization step to the mass of cellulose in the powder pulp is 2.0 to 3.5.

3.  The method according to claim 1 or 2, wherein the solubilization step is performed at a mixing temperature of 30 to 40°C.

4.  Low-substituted hydroxypropyl cellulose that is in a form of particles, comprising:

    fine particles having a length of fiber of less than 40 $\mu$m;
    spherical particles having a length of 40 $\mu$m or more and consisting of

    first spherical particles having an elongation, which is a ratio of a diameter of fiber to a length of fiber, of 0.5 or more, and
    second spherical particles having

    an elongation of less than 0.5,
    an aspect ratio, which is a ratio of a minimum Feret diameter to a maximum Feret diameter, of 0.5 or more, and
    a circularity, which is a ratio of a perimeter ($P_{EQPC}$) of a circle that has the same area as a projection area of a particle to a real perimeter ($P_{real}$) of the particle, of 0.7 or more; and

    fibrous particles consisting of

    long fibrous particles having a length of fiber of 200 $\mu$m or more and an elongation of less than 0.5, and consisting of

    first long fibrous particles having an aspect ratio of less than 0.5, and
    second long fibrous particles having an aspect ratio of 0.5 or more and a circularity of less than 0.7, and

    short fibrous particles having a length of fiber of 40 $\mu$m or more and less than 200 $\mu$m and an elongation of less than 0.5, and consisting of

first short fibrous particles having an aspect ratio of less than 0.5, and
second short fibrous particles having an aspect ratio of 0.5 or more and a circularity of less than 0.7,

wherein the low-substituted hydroxypropyl cellulose have

a hydroxypropoxy group content of 5 to 16% by mass,
a volume fraction of the spherical particles of 60 to 90% relative to all of the particles, and
a volume fraction ratio of the second spherical particles to the first spherical particles of 0.90 to 1.35, and

wherein said form of particles is classified into the fine particles, the spherical particles, and the fibrous particles on a basis of dynamic image analysis.

FIG.1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H11322802 A

**Non-patent literature cited in the description**

- **R. L. CARR**. *Chem. Eng*, 18 January 1965, vol. 72, 163
- **R. L. CARR**. *Chem. Eng*, 01 February 1965, 69
- **R. L. CARR**. *Chem.Eng*, 13 October 1969, vol. 76, 7
- An Illustrated Explanation of Powder Properties (revised and enlarged edition). Nikkei Technical Books. Japanese Society of Powder Technology and the Japanese Association of Powder Process Industry and Engineering, 1985, 151
- Loss on Drying test" in "General Tests. Japanese Pharmacopoeia